(19) Europäisches Patentamt
European Patent Office
Office européen des brevets

(11) EP 1 529 040 B1

(12) # EUROPÄISCHE PATENTSCHRIFT

(45) Veröffentlichungstag und Bekanntmachung des Hinweises auf die Patenterteilung:
**28.12.2005 Patentblatt 2005/52**

(21) Anmeldenummer: **03784025.3**

(22) Anmeldetag: **18.07.2003**

(51) Int Cl.[7]: **C07D 317/60**, A61K 31/36, A61P 11/06

(86) Internationale Anmeldenummer:
**PCT/EP2003/007821**

(87) Internationale Veröffentlichungsnummer:
**WO 2004/014888 (19.02.2004 Gazette 2004/08)**

(54) **ENDIANDRIC ACID H DERIVATE ALS C-MAF INHIBITOREN ZUR VERWENDUNG GEGEN ASTHMA**

ENDIANDRIC ACID H DERIVATIVES AS C-MAF INHIBITORS FOR USING AGAINST ASTHMA

DERIVES D'ACIDE ENDIANDRIQUE H EN TANT QU'INHIBITEURS DE C-MAF UTILISABLES CONTRE L'ASTHME

(84) Benannte Vertragsstaaten:
**AT BE BG CH CY CZ DE DK EE ES FI FR GB GR HU IE IT LI LU MC NL PT RO SE SI SK TR**

(30) Priorität: **02.08.2002 DE 10235624**

(43) Veröffentlichungstag der Anmeldung:
**11.05.2005 Patentblatt 2005/19**

(73) Patentinhaber: **Sanofi-Aventis Deutschland GmbH
65929 Frankfurt am Main (DE)**

(72) Erfinder:
- **EDER, Claudia
65719 Hofheim (DE)**
- **KOGLER, Herbert
61479 Glashütten (DE)**
- **HAAG-RICHTER, Sabine
60322 Frankfurt (DE)**
- **SINGLETON, Robert, Walker
Ringoes, NJ 08551 (US)**
- **SUBRAMANIAM, Arun
Lebanon, NJ 08833 (US)**

(56) Entgegenhaltungen:
- **BANFIELD J. E., ET AL.: "Constituents of Some Species of Beilschmiedia and Endiandra (Lauraceae): New Endiandric Acid and Benzopyran Derivatives Isolated from B. oligandra" AUST. J. CHEM., Bd. 47, 1994, Seiten 587-607, XP002123244 in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Asthma ist eine Erkrankung des Immunsystems, die sich zum Beispiel als Asthma bronchiale in Form von akut auftretender, anfallartiger Luftnot mit exspiratorischer Ventilationsbehinderung äußert. Bisher gibt es zur Behandlung von Asthma nur Medikamente, die die Symptome mildern, nicht aber solche, die hemmend in den Mechanismus, der für die Exprimierung von Entzündungsmediatoren verantwortlich ist, eingreifen. Diese Entzündungsmediatoren, insbesondere die Zytokine Interleukin-4 (IL-4), Interleukin-13 (IL-13) und Interleukin-5 (IL-5), sind bei der Asthmaerkrankung überexprimiert und verantwortlich für Entzündung, Eosinophilie, Schleimbildüng und bronchiale Hyperreaktivität, die die Krankheit aufrecht erhalten. Die Expression dieser Zytokine ist in den Lungen von Asthmatikern erhöht (Ray & Cohn, J. Clin. Invest. 1999, 104(8), 985-993). Studien mit transgenen Tieren zeigten, daß ein knock-out des IL-4 Gens die allergischen Entzündungsreaktionen reduziert. Kim et al. (Immunity 1999, 10, 745) haben gezeigt, daß der Transkriptionsfaktor c-maf für die Gewebespezifische Expression von IL-4 in einer Unterklasse von T-Helferzellen und somit für allergische Entzündungsreaktionen verantwortlich ist. c-maf gehört zur Maf-Familie, einer Familie von Leucin-haltigen Zipper-Proteinen, die in die Expressionsregulierung einer ganzen Reihe von Genen involviert ist. In TH2-Zellen, einer Untergruppe der CD4+ Helferzellen, bewirkt c-maf in Synergie mit NFAT (nuclear factor of activated T cells) eine Transaktivierung des IL-4 Promotors, was wiederum zu einer Erhöhung der Zytokin-Konzentration führt.

Ein Inhibitor von c-maf sollte daher die Zytokin-Konzentration senken, wobei zum einen ein niedrigerer IL-4 Level zu einer verminderten IgE (Immunglobulin E) Konzentration führt, da IL-4 entscheidend für die Stimulierung von B-Zellen zur Produktion von IgE ist. Eine Reduzierung von IgE wiederum würde eine verminderte Mastzell-Degranulation und eine verminderte Freisetzung von Histamin, Serotonin und anderen Entzündungsfaktoren zur Folge haben. Zum anderen beschreiben Ho et al. (J. Exp. Med., 1998, 10, 1859-1866), daß die Konzentration des TH2 Phänotyps auf autokrinem Weg durch IL-4 reguliert wird. Daher hat eine Senkung des IL-4 Spiegels eine Verringerung dieses T-Zelltyps zur Folge, was wiederum das Gleichgewicht zwischen TH1 und TH2 in Richtung TH1 verschiebt. Dies würde die IL-4 Konzentration weiter senken und brächte den zusätzlichen Vorteil einer Reduktion der IL-5 bzw. IL-13-Produktion, was eine Verminderung der Eosinophilie, Schleimbildung und bronchialer Hyperreaktivität zur Folge hätte.

Gene, die selektiv in TH2 Zellen exprimiert werden, sind daher bevorzugte Targets für eine therapeutische Anwendung um die entzündliche Komponente bei Asthma und Allergie selektiv zu beeinflussen.

In diesem Zusammenhang werden momentan IL-4 und IL-5 Antikörper in klinischer Prüfung erprobt (Foster et al., Trends Mol. Med., 2002, 8, 162). Man erwartet, dass selektive Therapeutika die weitverbreitete Therapie mit Glucocorticoiden ersetzen, die bisher die einzige Möglichkeit darstellt, die entzündlichen Reaktionen bei Asthma einzudämmen.

Eine tetrazyklische Verbindung der Formel

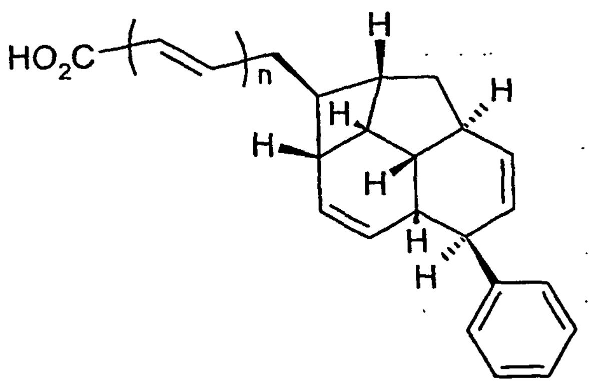

in der n = 0 ist, wird als Endiandric acid A bezeichnet, und in der n = 1 ist, als Endiandric acid B. Die Verbindungen sind erhältlich aus Extrakten der Pflanzenfamilie der Lauraceen, speziell der Gattungen *Endiandra* und *Beilschmiedia* (Bandaranayake et al., Aust. J. Chem., 1981, 34, 1655-67; Banfield et al., Aust. J. Chem., 1994, 47, 587-607).

Bandaranayake et al. beschreiben die Dihydro- und Tetrahydro-Derivate der Endiandric acid A, erhältlich durch partielle oder vollständige Umsetzung mit Wasserstoff in Gegenwart von Pd/C.

Banfield et al. beschreiben am Phenylrest durch eine Methylendioxy-Gruppe substituierte Endiandric acid A.

Bekannt sind darüber hinaus Derivate der Endiandric acid A und B, in denen die Säuregruppe umfunktionalisiert ist, beispielsweise als $CH_2OSiPh_2t$-Bu, $CH_2Br$, $CH_2CN$, verestert zu $CO_2CH_3$, oder reduziert zum Aldehyd CHO oder zum Alkohol $CH_2OH$ (Nicolaou et al., J. Am. Chem. Soc., 1982, 104, 5555-5557 und 5560-5562).

Endiandric acid Derivate, in denen die Doppelbindung im dem Phenylring benachbarten Ring in Konjugation

mit dem Phenylring ist, sind bisher nicht bekannt. Bandaranayake et al. beschreiben, daß entsprechende Isomefisierungsversuche fehlgeschlagen sind.

Die biologische Wirkung von Endiandric acid Derivaten wurde bisher nicht untersucht.

Aufgabe der vorliegenden Erfindung ist es, neuartige Endiandric acid Derivate bereitzustellen.

Es wurde überraschend gefunden, daß die afrikanische Pflanze *Beilschmiedia fulva* hoch wirksame neuartige Verbindungen zu bilden vermag, die als c-maf-Inhibitor wirksam sind.

Die vorliegende Erfindung betrifft daher eine Verbindung der Formel (I)

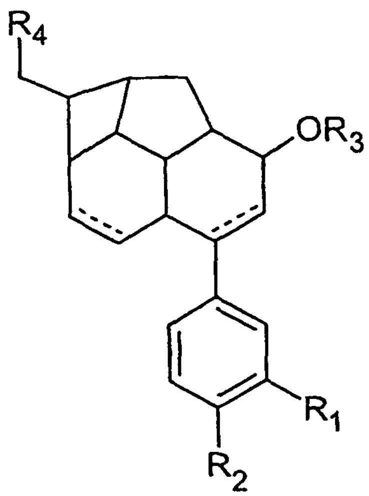

(I)

wobei

$R_1$ und $R_2$ unabhängig voneinander
0.0 H oder
1.0 eine -O-$C_1$-$C_6$-Alkyl-, -O-$C_2$-$C_6$-Alkenyl-, -O-$C_2$-$C_6$-Alkinyl- oder -O-$C_6$-$C_{10}$-Aryl-Gruppe sind, worin Alkyl, Alkenyl und Alkinyl geradkettig oder verzweigt sind, und worin die Alkyl-, Alkenyl- und Alkinyl-Gruppen gegebenenfalls ein- oder zweifach substituiert sind durch:

2.1 -OH,
2.2 =O,
2.3 -O-$C_1$-$C_6$-Alkyl, worin Alkyl geradkettig oder verzweigt ist,
2.4 -O-$C_2$-$C_6$-Alkenyl, worin Alkenyl geradkettig oder verzweigt ist,
2.5 - $C_6$-$C_{10}$-Aryl,
2.6 -NH-$C_1$-$C_6$-Alkyl, worin Alkyl geradkettig oder verzweigt ist,
2.7 -NH-$C_2$-$C_6$-Alkenyl, worin Alkenyl geradkettig oder verzweigt ist,
2.8 -$NH_2$ oder
2.9 Halogen,

und worin die Aryl-Gruppe gegebenenfalls ein- oder zweifach substituiert ist durch Substituent 2.1 oder 2.3 bis 2.9, worin die Substituenten 2.3, 2.4, 2.6 und 2.7 weiter substituiert sein können durch -CN, -Amid oder -Oxim-Funktionen, und 2.5 weiter substituiert sein kann durch -CN oder Amid-Funktionen, oder
$R_1$ und $R_2$ bilden zusammen einen Ring, wobei $R_1$ und $R_2$ eine Gruppe -O-[($C_1$-$C_6$)-Alkylen]-O- bedeuten,
$R_3$
1.0 H oder
2.0 eine $C_1$-$C_6$-Alkyl-, $C_2$-$C_6$-Alkenyl-, $C_2$-$C_6$-Alkinyl- oder $C_6$-$C_{10}$-Aryl-Gruppe sind, worin Alkyl, Alkenyl und Alkinyl geradkettig oder verzweigt sind, und worin die Alkyl-, Alkenyl- und Alkinyl-Gruppen gegebenenfalls ein- oder zweifach substituiert sind durch:

2.1-OH,
2.2 =O,
2.3 -O-$C_1$-$C_6$-Alkyl, worin Alkyl geradkettig oder verzweigt ist,

2.4 $-O-C_2-C_6$-Alkenyl, worin Alkenyl geradkettig oder verzweigt ist,
2.5 - $C_6-C_{10}$-Aryl,
2.6 $-NH-C_1-C_6$-Alkyl, worin Alkyl geradkettig oder verzweigt ist,
2.7 $-NH-C_2-C_6$-Alkenyl, worin Alkenyl geradkettig oder verzweigt ist,
2.8 $-NH_2$ oder
2.9 Halogen,

und worin die Aryl-Gruppe gegebenenfalls ein- oder zweifach substituiert ist durch Substituent 2.1 oder 2.3 bis 2.9, worin die Substituenten 2.3, 2.4, 2.6 und 2.7 weiter substituiert sein können durch -CN, -Amid oder -Oxim-Funktionen, und 2.5 weiter substituiert sein kann durch -CN oder Amid-Funktionen,

$R_4$

$CO_2R_3$, $CO_2NHR_3$, CHO, $CH_2OR_3$, $CH_2OSi(R_3)_3$, $CH_2Br$, $CH_2CN$ bedeutet, wobei $R_3$ wie oben definiert ist,

oder eine stereoisomere Form der Verbindung der Formel (I) oder ein physiologisch verträgliches Salz der Verbindung der Formel (I) oder ein Salz einer stereoisomeren Form der Verbindung der Formel (I).

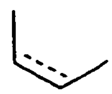

oder

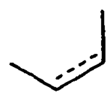

bedeutet unabhängig voneinander eine Einfachbindung oder eine Doppelbindung.

Vorzugsweise betrifft die Erfindung eine Verbindung der Formel (II),

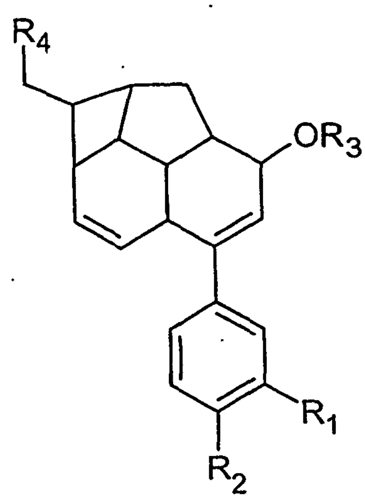

wobei die Reste $R_1$ bis $R_4$ wie oben definiert sind.

$C_1-C_6$-Alkyl bedeutet ein geradkettiges oder verzweigtes Alkyl mit 1 bis 6 C-Atomen, bevorzugt mit 1 bis 4 C-Atomen, z.B. Methyl, Ethyl, i-Propyl, tert.Butyl und Hexyl.

$C_2-C_6$-Alkenyl bedeutet ein geradkettiges oder verzweigtes Alkenyl mit 2 bis 6 C-Atomen, das einfach, zweifach oder dreifach ungesättigt ist, z.B. Allyl, Crotyl, 1-Propenyl, Penta-1,3-dienyl und Pentenyl.

$C_2-C_6$-Alkinyl bedeutet ein geradkettiges oder verzweigtes Alkinyl mit 2 bis 6 C-Atomen, das einfach oder zweifach ungesättigt ist, z.B. Propinyl, Butinyl und Pentinyl.

$C_6-C_{10}$-Aryl bedeutet eine Arylgruppe mit 6 bis 10 C-Atomen, z.B. Phenyl, Benzyl oder 1- oder 2- Naphthyl, die auch noch substituiert sein kann, beispielsweise durch Halogen, wie Chlor, Brom, Fluor, durch Alkyl mit 1-4 C-Atomen, vorzugsweise Methyl-, durch Hydroxy, durch Alkoxy mit 1-4 C Atomen, insbesondere Methoxy oder durch Trifluormethyl stehen.

$C_1-C_6$-Alkylen bedeutet eine Alkylengruppe mit 1 bis 6 C-Atomen, bevorzugt mit 1 bis 4 C-Atomen, z.B. Methylen, Ethylen, i-Propylen, tert.Butylen und Hexylen.

$R_1$ und $R_2$ sind vorzugsweise H oder eine Gruppe $-O-[(C_1-C_6)-Alkyl]-O-$, besonders bevorzugt $-OCH_2-O-$.

$R_3$ ist vorzugsweise H.

$R_4$ ist bevorzugt $COOR_3$, wobei $R_3$ vorzugsweise H.

Besonders bevorzugt betrifft die Erfindung eine Verbindung der Formel (III)

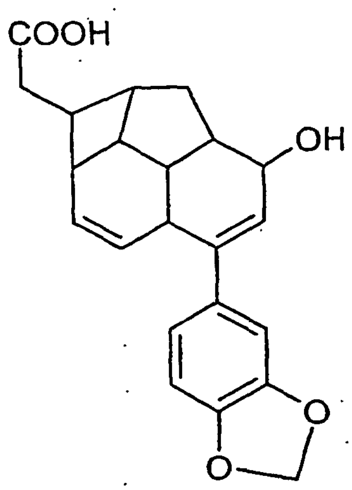

(III)

Ferner besonders bevorzugt betrifft die Erfindung eine Verbindung der Formel (IV), die im folgenden als Endiandric acid H bezeichnet wird:

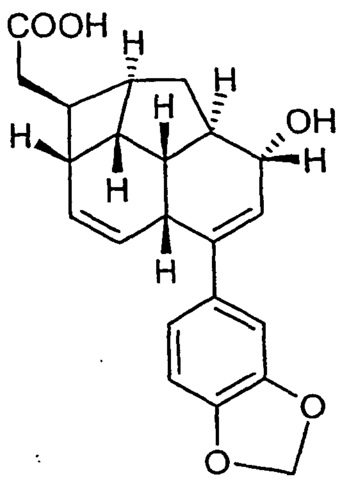

(IV)

Die erfindungsgemäßen Verbindungen der Formel (I) enthalten ein tetrazyklisches Ringsystem mit einem Phenylrest, der im Gegensatz zu den bisher beschrieben Verbindungen in Konjugation mit einer der beiden Doppelbindungen im tetrazyklischen Ringsystem steht, und weisen eine zusätzliche Hydroxylgruppe auf. Aufgrund ihrer abweichenden chemischen Struktur besitzten die Verbindungen der Formel (I) neue physiko-chemische, biologische und pharmakologische Eigenschaften.

Die Erfindung betrifft ferner eine Verbindung erhältlich aus der Pflanze *Beilschmiedia fulva,* PLA 101037, oder Zellkulturen der Pflanze *Beilschmiedia fulva,* PLA 101037, charakterisiert durch die Summenformel $C_{22}H_{21}O_5$, durch die $^1$H-NMR chemischen Verschiebungen $\delta$ = 1.733, 1.801, 1.209, 1.851, 1.946, 2.339, 2.354, 2.400 2.440, 2.765, 3.351, 4.000, 5.207, 5.593, 5.845, 6.00, 6.01, 6.873, 6.908, 6.995 und durch die $^{13}$C-NMR chemischen Verschiebungen $\delta$ = 33.62, 33.76, 34.82, 34.99, 40.32, 40.61, 40.69, 40.90, 44.02, 71.96, 100.92, 106.66, 108.10, 119.86, 126.33, 127.59, 131.41, 133.76, 140.15, 146.48, 147.51 173.47.

Die Erfindung betrifft ferner ein Verfahren zur Herstellung der Verbindung der Formel (I), dadurch gekennzeichnet, daß

1. die Pflanze *Beilschmiedia fulva,* PLA 101037, oder Zellkulturen der Pflanze *Beilschmiedia fulva,* PLA 101037, unter geeigneten Bedingungen extrahiert wird,
2. die Verbindung der Formel (IV) isoliert wird, und
3. gegebenenfalls zu einer Verbindung der Formel (I) derivatisiert und/oder zu einem physiologisch verträglichen Salz der Verbindung der Formel (I) umgesetzt wird.

Die Erfindung betrifft ferner ein Verfahren zur Herstellung einer Verbindung der Formel (IV), dadurch gekennzeichnet, daß

1. die Pflanze *Beilschmiedia fulva,* PLA 101037, oder Zellkulturen der Pflanze *Beilschmiedia fulva,* PLA 101037, unter geeigneten Bedingungen extrahiert wird,
2. die Verbindung der Formel (IV) isoliert wird, und
3. gegebenenfalls zu einem physiologisch verträglichen Salz der Verbindung der Formel (IV) umgesetzt wird.

Für die Extraktion der Verbindung der Formel (IV) aus der Pflanze *Beilschmiedia fulva* oder eine ihrer Varianten oder Mutanten wird diese zunächst unter geeigneten Bedingungen gezüchtet, bis sich Endiandric acid H der Formel (IV) in dem Pflanzenmaterial anhäuft. Vorzugsweise wird die Pflanze *Beilschmiedia fulva,* ihre Mutanten und/oder Varianten auf geeigneten Böden in tropischem oder subtropischem Klima gezüchtet. Die Pflanzenproduktion wird besonders bevorzugt unter tropischen Bedingungen durchgeführt, beispielsweise bei einer Temperatur zwischen 18 und 35°C und bei einer Luftfeuchtigkeit von größer oder gleich 70%, vorzugsweise von 70-90%.

Für die Extraktion der Verbindung der Formel (IV) aus lebenden Zellen der Pflanze *Beilschmiedia fulva* werden diese zunächst in eine geeignete Nährlösung übertragen und gezüchtet, bis sich in dem Medium die erfindungsgemäße Verbindung der Formel (IV) anhäuft. Die Zellkulturen werden vorzugsweise aus Kalluskulturen angelegt. Die Nährmedien bestehen neben Mineralien und Vitaminen auch aus mindestens einer Kohlenstoff-Quelle, zum Beispiel Saccharose, und mindestens einer Stickstoff-Quelle, wie zum Beispiel einem Nitrat- oder Ammonium-Salz.

*Beilschmiedia fulva* ist ein Baum aus der Familie der Lauraceae. In der Familie der Lauraceae finden sich viele immergrüne tropische Gewürz- und Nutzpflanzen. Das Verbreitungsgebiet der Lauraceae sind die gesamten Tropen: Die Probe der Pflanze *Beilschmiedia fulva,* PLA 101037, wurde in Gabun, speziell in der Gegend um la Makandé im Bereich der Baumkrone des Regenwaldes gesammelt. Die Probe wurde in unmittelbarer Nähe der Makande Research Field Station (Koordinaten 0° 40' 860" S - 11 ° 54' 750" E) gesammelt. Bei *Beilschmiedia fulva* handelt es sich um einen Baum, der bis zu 30 m hoch werden kann und somit Bestandteil der Baumkronenregion des Regenwaldes ist. Die Blätter sind blau-grün, die Früchte rot. Der an der Basis zylindrisch geformte Stamm ist mit wenig Rinde von spröder Konsistenz bedeckt und weist eine braun-rötliche Färbung auf. Die Rindenschicht ist etwa 4-5 mm dick und hat einen charakteristischem Geruch.

Zur Isolierung von Endiandric acid H können auch andere Arten aus der Gattung *Beilschmiedia* verwendet werden, bzw. auch Pflanzen derselben Spezies, die von einem anderen Standort stammen. Der Gehalt an Endiandric acid H kann je nach Standortverhältnissen, wie z.B. Bodenbeschaffenheit, Temperatur, Feuchtigkeit, Lichteinfall variieren.

Das erfindungsgemäße Verfahren kann für die Extraktion und Isolierung in einem großen Bereich von zu extrahierendem Pflanzenmaterial eingesetzt werden, beispielsweise im Labormaßstab (100 g bis 1 kg trockenes Pflanzenmateria) bis zum industriellen Maßstab (100 bis > 1000 kg).

Die Kultivierung der Pflanze *Beilschmiedia fulva* kann im Freiland oder vorzugsweise im Gewächshaus erfolgen.

Alternativ können pflanzliche Zellkulturen von *Beilschmiedia fulva* zur Produktion der erfindungsgemäßen Verbindungen eingesetzt werden. In der Regel kultiviert man dazu in mehreren Stufen, d.h. man stellt zunächst eine oder mehrere Vorkulturen in einem geeigneten Flüssigmedium her, mit denen dann die Hauptkultur beimpft werden kann. Das Ausgangsmaterial besteht in der Regel aus Kalluskulturen. Durch die Auswahl geeigneter Bioreaktoren zur Züchtung der pflanzlichen Zellkultur kann eine optimale Durchmischung und Belüftung der Kultur ohne die Einwirkung von zu starken Scherkräften auf die Pflanzenzellen und damit optimales Zellwachstum und MetabolitProduktion erzielt werden. Beispielsweise können Airlift- oder Blasensäulenreaktoren, sowie Blatt- oder Propellerrührwerke zur Durchmischung der Kulturen eingesetzt werden. Die Zellen können als Einzelzellen bzw. verzweigte oder unverzweigte Zell-Aggregate oder-ketten wachsen. Die Metabolitproduktion kann durch Stimulation mit exogenen Faktoren, z.B. Schwermetallsalzen oder pflanzlichen Elicitoren induziert werden.

Die Produktbildung in der Pflanzenzellkultur kann anhand des pH-Wertes der Kulturen sowie durch chromatographische Methoden, wie zum Beispiel Dünnschichtchromatographie, HPLC oder Ausprüfen der biologischen Aktivität überwacht werden. Endiandric acid H der Formel (IV) kann neben der Rinde auch in anderen Pflanzenteilen enthalten sein.

Die Isolierung und Aufreinigung der erfindungsgemäßen Endiandric acid H der Formel (IV) aus der Pflanze

oder dem Kulturmedium erfolgt nach bekannten Methoden unter Berücksichtigung der chemischen, physikalischen und biologischen Eigenschaften der Naturstoffe. Zum Testen der Konzentration der erfindungsgemäßen Verbindungen im Ausgangsmaterial oder in den einzelnen Isolierungsstufen kann die HPLC verwendet werden, wobei die Menge der gebildeten Substanz zweckmäßig mit einer Eichlösung verglichen wird.

Zur Isolierung der erfindungsgemäßen Endiandric acid H der Formel (IV) wird die Pflanze *Beilschmiedia fulva* geerntet, wobei zunächst die Blätter, Stengel, Holz, die Rinde oder Wurzeln gesammelt und nach Pflanzenteilen getrennt, noch in frischem Zustand oder getrocknet und anschließend aus dem Pflanzenmaterial mit einem gegebenenfalls wasserhaltigem organischen Lösungsmittel extrahiert. Vorzugsweise wird zur Isolierung der erfindungsgemäßen Verbindungen die Rinde extrahiert.

Die Extrakte werden vereinigt, mit Wasser verdünnt und mit einem geeigneten, mit Wasser nicht mischbarem organischen Lösungsmittel, zum Beispiel mit n-Butanol, extrahiert. Die anschließend abgetrennte organische Phase wird gegebenfalls im Vakuum konzentriert. Man kann das Konzentrat zum Entfetten des Wertproduktes mit einem unpolaren Lösungsmittel, in dem die erfindungsgemäßen Endiandric acid H sehr wenig löslich sind, wie zum Beispiel mit Hexan, Petroleumether, Diethylether verdünnen. Hierbei präzipitiert die Endiandric acid H, die lipophilen Verunreinigungen bleiben gelöst und werden durch übliche Fest/Flüssig-Phasentrennungen entfernt. Der Niederschlag, der die Endiandric acid H enthält, wird lyophilisiert. Das Lyophilisat wird weiter aufgereinigt.

Die weitere Aufreinigung von Endiandric acid H erfolgt durch Chromatographie an geeigneten Materialien, vorzugsweise z.B. an Molekularsieben, an Normal-Phasenträger, wie z. B. Kieselgel, Aluminiumoxid, an Ionenaustauschern oder an Adsorberharzen bzw. an Umkehr-Phasen (reversed phase, RP). Mit Hilfe dieser Chromatographie wird die Endiandric acid H abgetrennt. Die Chromatographie der Endiandric acid H erfolgt mit gepufferten wässrigen Lösungen oder Gemischen von wässrigen und organischen Lösungen.

Unter Gemischen von wässrigen und organischen Lösungen versteht man alle mit Wasser mischbaren organischen Lösemittel, vorzugsweise Methanol, 2-Propanol und Acetonitril, in einer Konzentration von 10 bis 90 % Lösemittel, vorzugsweise 15 60 % Lösemittel oder auch alle gepufferten wässrigen Lösungen, die mit organischen Lösemitteln mischbar sind.

Die Abtrennung der Endiandric acid H erfolgt mit Hilfe der Reversed Phase Chromatographie, beispielsweise an MCI® (Adsorberharz von Mitsubishi, Japan) oder Amberlite XAD® (TOSOHAAS), an weiteren hydrophoben Materialien, wie zum Beispiel an RP-8- oder RP-18-Phasen oder an Polyamiden. Außerdem kann die Trennung mit Hilfe von Gel-Chromatographie oder der Normalphasen-Chromatographie, zum Beispiel an Kieselgel oder Aluminiumoxyd, erfolgen.

Die Chromatographie der Endiandric acid H erfolgt mit gepufferten oder angesäuerten wäßrigen Lösungen oder Gemischen von wäßrigen Lösungen mit Alkoholen oder anderen, mit Wasser mischbaren organischen Lösemitteln. Als organisches Lösemittel wird vorzugsweise Propanol und Acetonitril verwendet.

Unter gepufferten oder angesäuerten wäßrigen Lösungen versteht man z.B. Wasser, Phosphatpuffer, Citratpuffer, Ammoniumacetat in einer Konzentration von 1 mM bis 0,5 M, vorzugsweise 10 mM, sowie Ameisensäure, Essigsäure, Trifluoressigsäure oder allen handelsüblichen, dem Fachmann bekannten Säuren, vorzugsweise in einer Konzentration von 0,01 bis 3 %.

Chromatographiert wird mit einem Gradienten, der mit 100 % wäßrigem Puffer beginnt und mit 100 % Lösemittel endet, vorzugsweise wird ein linearer Gradient von 10 bis 60 % 2-Propanol oder Acetonitril gefahren.

Die Gelchromatographie wird an Polyacrylamid- oder Mischpolymergelen durchgeführt, wie z.B. Biogel-P 2® (Fa. Biorad), Fractogel TSK HW 40® (Fa. Merck, Deutschland oder Toso Haas, USA) oder an Sephadex® (Fa. Pharmacia, Uppsala, Schweden).

Die Reihenfolge der vorgenannten Chromatographien ist umkehrbar.

Ein weiterer Reinigungsschritt für die erfindungsgemäßen Verbindungen ist die Kristallisation. Beispielsweise kristallisiert Endiandric acid H der Formel (IV) aus organischen Lösungsmitteln und aus Gemischen von Wasser mit organischen Lösungsmitteln. Die Kristallisation wird in an sich bekannter Weise zum Beispiel durch Konzentrieren oder Abkühlen gesättigter Lösungen durchgeführt.

Endiandric acid H der Formel (IV) ist im festen Zustand und in Lösungen im pH-Bereich zwischen 3 und 8, insbesondere 4 und 6, stabil.

Die Verbindung der Formel (I) kann nach an sich bekannten Methoden derivatisiert werden (J. March, Advanced Organic Synthesis, 4$^{th}$ Edition, John Wiley & sons., 1992). Beispielsweise kann die Carboxylfunktion verestert oder zum primären Alkohol reduziert werden, oder sie kann zu einem Amid umgewandelt werden. Die Reduktion von Carbonyl-Gruppen kann mit Metall-Hydriden, wie Aluminium-Hydriden oder Bor-Hydriden erfolgen. Eine Reduktion zu den gesättigten Verbindungen kann zum Beispiel mit Wasserstoff in Gegenwart von geeigneten Katalysatoren erreicht werden. Die Hydroxyfunktion kann verethert werden.

Verbindungen der Formel (I) können nach dem Fachmann bekannten Methoden in deren physiologisch verträgliche Salze übergeführt werden.

Unter physiologisch verträglichen Salzen von Verbindungen der Formel (I) versteht man sowohl deren organische als auch anorganische Salze, wie sie in Remington's Pharmaceutical Sciences (17. Auflage, Seite 1418 (1985)) beschrieben sind. Aufgrund der physikalischen und chemischen Stabilität und der Löslichkeit sind für saure Gruppen unter anderem Natrium-, Kalium-, Calcium-und Ammoniumsalze bevorzugt. Für basische Gruppen sind unter anderem Salze der Salzsäure, Schwefelsäure, Phosphorsäure oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Die vorliegende Erfindung umfaßt alle stereoisomeren Formen der Verbindungen der Formel (I). Zur Erfindung gehören alle möglichen Enantiomeren und Diastereomeren, ebenso Mischungen von zwei oder mehr stereoisomeren Formen, zum Beispiel Mischungen von Enantiomeren und/oder Diastereomeren, in allen Verhältnissen. Enantiomere sind in enantiomerenreiner Form, sowohl als linksdrehende als auch als rechtsdrehende Antipoden, R- und S-Konfigurationen, in Form von Racematen und in Form von Mischungen der beiden Enantiomeren in allen Verhältnissen Gegenstand der Erfindung. Bei Vorliegen einer cis/trans-Isomerie sind sowohl die cis-Form als auch die trans-Form und Gemische dieser Formen in allen Verhältnissen Gegenstand der Erfindung.

Aufgrund ihrer pharmakologischen Eigenschaften eignen sich die erfindungsgemäßen Verbindungen der Formel (I) zur Anwendung als Arzneimittel in der Human- und/oder Tiermedizin.

Die Erfindung betrifft daher ferner ein Arzneimittel mit einem Gehalt an mindestens einer Verbindung der Formel (I) und einem oder mehreren physiologisch geeigneten Hilfsstoffen.

Die erfindungsgemäßen Arzneimittel werden im allgemeinen oral, lokal oder parenteral verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei deren Herstellung üblicherweise physiologisch geeignete Hilfsmittel wie Träger-, Spreng-, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z.B. Magnesiumcarbonat, Titandioxid, Laktose, Mannit und andere Zucker, Talkum, Milcheiweiß, Gelatine, Stärke, Vitamine, Cellulose und ihre Derivate, tierische oder pflanzliche Öle, Polyethylenglykole und Lösungsmittel, wie etwa steriles Wasser, Alkohole, Glycerin und mehrwertige Alkohole genannt.

Gegebenenfalls können die Dosierungseinheiten für die orale Verabreichung mikroverkapselt werden, um die Abgabe zu verzögern oder über einen längeren Zeitraum auszudehnen, wie beispielsweise durch Überziehen oder Einbetten des Wirkstoffs in Teilchenform in geeignete Polymere, Wachse oder dergleichen.

Vorzugsweise werden die pharmazeutischen Präparate in Dosierungseinheiten hergestellt und verabreicht, wobei jede Einheit als aktiven Bestandteil eine bestimmte Dosis mindestens einer Verbindung der Formel (I) enthält. Bei festen Dosierungseinheiten wie Tabletten, Kapseln und Suppositorien kann diese Dosis bis zu 1 g, bevorzugt etwa 0,1 bis 200 mg, und bei Injektionslösungen in Ampullenform bis zu 1 g, vorzugsweise etwa 0,1 bis 100 mg, betragen.

Die zu verabreichende Tagesdosis ist abhängig vom Körpergewicht, Alter, Geschlecht und Zustand des Säugers. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber in mehreren kleineren Dosierungseinheiten als auch durch Mehrfachgabe unterteilter Dosen in bestimmten Intervallen erfolgen.

Die erfindungsgemäßen Arzneimittel werden dadurch hergestellt, daß man ein oder mehrere Verbindungen der Formel (I) mit einem oder mehreren physiologisch geeigneten Hilfsstoffen in eine geeignete Darreichungsform bringt.

Die Erfindung betrifft ferner die Verwendung einer Verbindung der Formel (I) zur Herstellung eines Arzneimittels, insbesondere zur Behandlung von allergischen Erkrankungen, von asthmatischen Erkrankungen, von entzündlichen Begleitsymptomen von Asthma und/oder von Krankheiten, die durch Hemmen von c-maf und NFAT behandelt werden können.

Das zipper-Protein c-maf stellt das Testtarget dar. Es spielt eine wichtige Rolle bei der Freisetzung von Entzündungsmediatoren, vor allem IL-4, und damit bei der Manifestation entzündlicher Symptome bei Asthma und Allergien. c-maf ist somit ein wichtiges therapeutisches Zielmolekül für Asthma, insbesondere wenn es allergisch bedingt ist. Im Test wird anhand der Transkriptionsrate von IL-4 die Aktivität von c-maf gemessen. Verbindungen, die mit der Bindung der beiden Transkriptionsfaktoren c-maf und NFAT interferieren, führen dabei zu einer verminderten Expression von Luciferase (read-out) durch eine Unterdrückung der Transkription eines humanen IL-4 Promotor/Luciferase-Konstrukts.

Die folgenden Beispiele sollen der näheren Erläuterung der Erfindung dienen, ohne die Breite der Erfindung in irgendeiner Weise begrenzen zu wollen. Prozentangaben beziehen sich auf das Gewicht. Mischungsverhältnisse bei Flüssigkeiten beziehen sich auf das Volumen, wenn keine anderen Angaben gemacht wurden.

Beispiele

Beispiel 1: Pflanzenproduktion (Sammeln der Samen, Aussat sowie Wachstums-und Ernte-Bedingungen).

Samen von *Beilschmiedia fulva* wurden nach der Reifung gesammelt und zur weiteren Anzucht der Pflanzen im Gewächshaus ausgesät. Die optimale Temperatur betrug ca. 28° C bei einer Luftfeuchtigkeit von 70-90%. Die Pflanzen wurden mehrere Monate bis Jahre kultiviert, bis zur Ernte der Rinde und anderer geeigneter Pflanzenteile.

Beispiel 2: Herstellen eines Primärextraktes von *Beilschmiedia fulva.*

Stücke der Rinde von *Beilschmiedia fulva* wurden im frischen Zustand gesammelt und anschließend bei ca. 40°C luftgetrocknet. Nach dem Trocknen wurden 100 g Trockenmaterial gemahlen und mit 1 L Methanol bei 40°C für 8h unter Rühren extrahiert. Nach Beenden der Extraktion wurden die Pflanzenreste abfiltriert und der methanolische Extrakt bis nahezu zur Trockene unter Vakuum eingeengt. Der Rückstand wurde mit etwas Wasser nochmals resuspendiert und anschließend gefriergetrocknet. Der so entstandene Primärextrakt konnte bei +4°C bis -20°C aufbewahrt werden oder zur weiteren Isolierung wie in Beispiel 3 verwendet werden. Zum Testen der biologischen Aktivität wurden aus dem Primärextrakt mittels Chromatographie an Polyamid und an Polystyrol-Adsorberharz Tannine und andere stark hydrophile bzw. lipophile Störsubstanzen entfernt.

Beispiel 3: Isolierung von Endiandric acid H aus der Pflanze *Beilschmiedia fulva.*

100 g getrocknete Rindenstücke von *Beilschmiedia fulva* werden entsprechend Beispiel 2 geerntet, in einer Mühle zerkleinert, mit 1 L Methanol 16 Stunden gerührt und anschließend filtriert. Die wirkstoffhaltige, methanolische Lösung wird im Vakuum konzentriert; die Trockenmasse beträgt 7,0 g. Das Konzentrat wird auf eine vorbereitete Glassäule (BPG 100, 4 L Innenvolumen, Fa. Pharmacia Biotech) aufgetragen, die mit ca. 0,5 L MCI-Gel® CHP-20P Material (Adsorberharz der Fa. Mitsubishi Chemicals, Japan) gefüllt ist. Eluiert wird mit einem Gradienten von 100% Wasser nach 100% Acetonitril. Der Säulendurchfluß (50 mL pro Minute) wird fraktioniert aufgefangen (je 50 mL) und im Biotest aktiven Fraktionen (Fraktion 18-21) zusammengefaßt. Konzentrieren im Vakuum und Gefriertrocknung ergeben 102 mg hellbraunes Pulvers.

Beispiel 4: Reinigung von Endiandric acid H mittels Reversed Phase HPLC.

Die 102 mg des nach Beispiel 3 gewonnen Pulvers wurden auf einer LUNA® 10 µ C18 (2) Säule (Größe: 21,2 mm x 250 mm, Fa. Phenomenex, Deutschland) aufgetragen und mit einem Gradienten von 3% bis 60% Acetonitril in 0,1% Ammoniumacetat/Wasser über 60 Minuten chromatographiert. Der Durchfluß des Elutionsmittels beträgt 33 ml/min, die Fraktionsgröße 33 ml. In den Fraktionen 24 und 25 befindet sich die Endiandric acid H. Die Lyophilisierung der genannten Fraktionen ergibt 3,7 mg Reinsubstanz (Reinheit > 95%).

Beispiel 5: Charakterisierung der Endiandric acid H.

Aussehen: in polaren organischen Lösungsmitteln lösliche, jedoch in Wasser nur wenig lösliche, weiße Substanz.
UV-Maxima (in Wasser/Acetonitril): 206, 262, 296. Durch hochauflösende Massenspektrometrie wurde für $(M + H)^+$ folgendes gefunden: 365.1392 amu. Dies entspricht einer Summenformel für die Endiandric acid H von $C_{22}H_{21}O_5$.
Durch Elektronenspray - Ionisation (ESI, positiv) werden mittels MS/MS-Fragmentierung folgende Ionen erhalten: 349 amu ($M+H-H_2O$) zu 331 amu ($-H_2O$), 303 amu ($-CH_2O_2$), 289 amu ($-C_2H_4O_2$).
Durch Elektronenspray - Ionisation (ESI, negativ) werden mittels MS/MS-Fragmentierung folgende Ionen erhalten: 365 amu $(M-H)^-$ zu 321 amu ($-CO_2$), 267 amu ($-C_5H_6O_2$) 227 amu ($-C_8H_{10}O_2$), 215 amu ($-C_9H_{10}O_2$) und kleineren Fragmente.

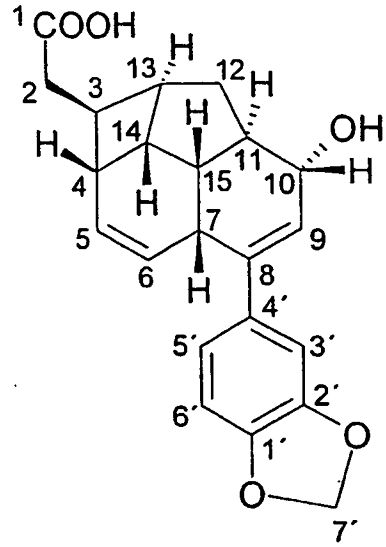

Tabelle 1:

| NMR-chemische Verschiebungen und Kopplungskonstanten von Endiandric acid H, DMSO, 303 K. | | | | | | |
|---|---|---|---|---|---|---|
| Pos. | δ ($^{13}C$) | m ($^{13}C$) | δ ($^{1}H$) | m ($^{1}H$) | $^{n}J_{CH}$ | $^{n}J_{HH}$ (J/Hz) |
| 1 | 173.47 | s | - | - | 2.44, 2.40 | - |
| 2 | 40.69 | t | 2.440, 2.400 | dd dd | 1.73,2.35 | 1.73 (8.2), 2.40 (12.2) 1.73 (7.7), 2.44 (12.2) |
| 3 | 40.90 | d | 1.733 | quint | 2.77, 2.44, 2.40, 1.80, 1.20, (5.59) | 2.44 (8.2), 2.40 (7.70), 2.35 (8), 2.34 (8) |
| 4 | 33.76 | d | 2.354 | | 5.204, (5.593), 2.765, 2.44, 2.40 | 1.73 (8), 2.77 (8), 5.59 (3.5), 5.21 (1) |
| 5 | 127.59 | d | 5.593 | dt | 1.733,2.765 | 5.21 (10.2), 3.35 (3.5), 2.35 (3.5) |
| 6 | 126.33 | d | 5.207 | dq | 1.851 | 5.59 (10.2), 5.845 (1), 2.35 (1), 3.35 (1) |
| 7 | 34.82 | d | 3.351 | m | 5.20, (5.59), 5.86, 1.85, 1.95 | 5.85 (1), 5.59 (3.5), 5.21 (<1), 4.00 (<1), 2.34 (1), 1.85 (5) |
| 8 | 140.15 | s | - | - | 6.91, 7.00, 5.21, 4.00 | - |
| 9 | 131.41 | d | 5.845 | q | 4.00, (1.95), (1.21) | 3.35 (1), 4.00 (1), 5.21 (1) |
| 10 | 71.96 | d | 4.000 | ddd | 1.21, 1.85, 1.95 | 1.95 (9), 3.35 (<1), 5.85 (1) |
| 11 | 44.02 | d | 1.946 | ddt | 5.85, 4.00, 2.77, 1.21, 1.80, 1.85, 5.59 | 4.00 (9), 1.21 (11.6), 1.80 (4), 1.85 (5) |
| 12 | 34.99 | t | 1.801 1.209 | dd dt | 4.00, 2.76, 1.73 2.34 | 1.21 (11), 1.95 (4) 1.80 (11), 1.95 (11.6), 2.34 (6) |
| 13 | 40.61 | d | 2.339 | | 2.44, 2.40, 1.21, 1.80, 1.73 | 2.77 (8),1.73 (8), 1.80 (<1), 1.21 (6) |
| 14 | 33.62 | d | 2.765 | q | (5.59), 2.34, 2.35, 1.80, 1.85 | 1.85 (8), 2.35 (8), 2.34 (8) |

Tabelle 1: (fortgesetzt)

| NMR-chemische Verschiebungen und Kopplungskonstanten von Endiandric acid H, DMSO, 303 K. | | | | | | |
|---|---|---|---|---|---|---|
| Pos. | δ ($^{13}C$) | m ($^{13}C$) | δ ($^{1}H$) | m ($^{1}H$) | $^{n}J_{CH}$ | $^{n}J_{HH}$ (J/Hz) |
| 15 | 40.32 | d | 1.851 | dt | 1.95, 1.80, 5.21, 2.34, (2.77) | 2.77 (8), 3.35 (5), 1.95 (5) |
| 1' | 147.51 | s | - | - | 6.00, 6.87, (7.00) | - |
| 2' | 146.48 | s | - | - | 6.00, 7.00, 6.91, (6.87) | - |
| 3' | 108.10 | d | 6.873 | d | (7.00) | 6.91 (8.2) |
| 4' | 119.86 | d | 6.908 | dd | 7.00 | 6.87 (8.2), 7.00 (1.6) |
| 5' | 133.76 | s | - | - | 6.87, 5.85 | - |
| 6' | 106.66 | d | 6.995 | d | 6.908 | 6.81 (1.6) |
| 7' | 100.92 | t | 6.01 6.00 | s s | - | - |

Beispiel 6: Bioassay

Zellkultur:

Mit dem humanen IL-4 Promotor (-6635 bis +66) wurde mittels Klonierung ein Luciferase-Reportergen-Konstrukt hergestellt (IL-4 luc). In einen weiteren Vektor wurde die cDNA von full-length murine nuclear factor of activated T cells (NFAT) kloniert. CHO-K1 Zellen wurde anschließend mit beiden Vektoren (IL-4 luc/NFAT) mittels Elektroporation transfiziert. Durch einen G418 Selektionsprozess konnte eine monoklonale IL-4 luc/NFAT-Zelllinie erhalten werden. Im Anschluß wurde full-length murine c-maf cDNA in einen geeigneten Vektor kloniert und die generierte IL-4 luc/NFAT-Zelllinie damit co-transfiziert. Die entstandene monoklonale Zelllinie trägt alle drei Vektoren (c-maf/IL-4 luc/NFAT) und wurde für das Screening verwendet.

Die Zellen wurden in der logarithmischen Wachstumsphase bei 37°C, 5% $CO_2$ in Gewebekulturflaschen der Fläche 225 $cm^2$ in folgendem Medium kultiviert: Ham's F-12 Nutrient Mixture, ergänzt mit 10 % fötalem Kälberserum, 1% Antibiotikum/Antimykotikum, 300 µg/ml Geneticin und 300 µg/ml Zeocin.

Testdurchführung:

16 Stunden vor dem Assay wurden CHO-K1 Zellen mittels Trypsinisierung geerntet, einmal mit PBS w/o Ca und Mg gewaschen, resuspendiert in Ham's F-12 Medium, welches zusätzlich 10 % fötales Kälberserum, 1 % Antibiotikum/Antimykotikum, 0,001 % Pluronic, 300 µg/ml Geneticin und 300 µg/ml Zeocin enthielt, und anschliessend mittels eines Hämozytometers quantifiziert. Die Zellen wurden auf Mikrotiterplatten in einer Zellkonzentration von 2000 Zellen pro well in jeweils 2 µl Medium ausplattiert. Die Zellen werden über Nacht bei 37 °C und 5% $CO_2$ inkubiert.

Stammlösungen der Testsubstanzen waren in DMSO gelöst. Diese Stammlösungen wurden mit Ham's F-12 Medium, welches zusätzlich 10 % fötales Kälberserum, 1% Antibiotikum/Antimykotikum (Gibco BRL, Nr. 15240-062), 300 µg/ml Geneticin und 300 µg/ml Zeocin enthielt, zu unterschiedlichen Konzentrationen verdünnt. Die CHO-K1 Zellen wurden mit 1 µl der so hergestellten Testsubstanzlösungen versetzt und anschließend für 8 Stunden bei 37 °C und 5% $CO_2$ inkubiert. Die Endkonzentration von DMSO betrug dabei maximal 0,83 % pro well.

Nach der Inkubationszeit werden die Zellen mit jeweils 3 µl Bright-Glow™ Luciferase Assay Reagenz (Promega Corporation, Madison, USA) pro well versetzt. Die Platten werden dann 30 Minuten ins Dunkle gestellt und anschließend am CyBiTM Lumax Reader vermessen. Als Positivkontrolle wurde DMSO in einer Endkonzentration von 33,3 % mitgetestet. Zusätzlich wurden die Positivkontrollen auf eine Konzentration von 0,83% normalisiert.

Auswertung.

Jede Testplatte enthält 64 Negativkontrollen (wells ohne Substanzzusatz) und 64 Positivkontrollen (wells, in denen die Zellen mit 1µl DMSO abgetötet wurden).
Die Inhibierung wird folgendermaßen berechnet:

$$[1-(LCS_{Probe}-LCS_{posKontr})/(LCS_{negKontr}-LCS_{posKontr})] \times 100\ (\%)$$

Endiandric acid H zeigt im Bioassay einen $IC_{50}$-Wert von 1,5 µM.

**Patentansprüche**

1. Verbindung der Formel (I)

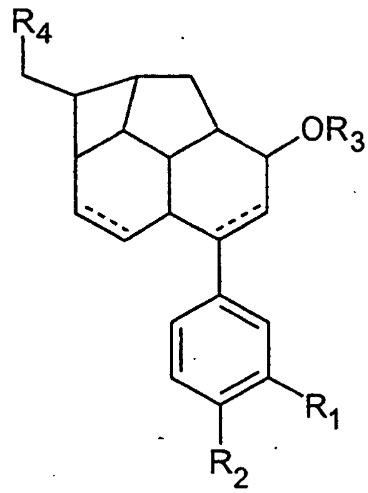

(I)

wobei

$R_1$ und $R_2$ unabhängig voneinander
1.0 H oder
2.0 eine -O-$C_1$-$C_6$-Alkyl-, -O-$C_2$-$C_6$-Alkenyl-, -O-$C_2$-$C_6$-Alkinyl- oder -O-$C_6$-$C_{10}$-Aryl-Gruppe sind, worin Alkyl, Alkenyl und Alkinyl geradkettig oder verzweigt sind, und worin die Alkyl-, Alkenyl- und Alkinyl-Gruppen; gegebenenfalls ein- oder zweifach substituiert sind durch:

2.1 -OH,
2.2 =O,
2.3 -O-$C_1$-$C_6$-Alkyl, worin Alkyl geradkettig oder verzweigt ist,
2.4 -O-$C_2$-$C_6$-Alkenyl, worin Alkenyl geradkettig oder verzweigt ist,
2.5 - $C_6$-$C_{10}$-Aryl,
2.6 -NH-$C_1$-$C_6$-Alkyl, worin Alkyl geradkettig oder verzweigt ist,
2.7 -NH-$C_2$-$C_6$-Alkenyl, worin Alkenyl geradkettig oder verzweigt ist,
2.8-$NH_2$ oder
2.9 Halogen,

und worin die Aryl-Gruppe gegebenenfalls ein- oder zweifach substituiert ist durch Substituent 2.1 oder 2.3 bis 2.9,
worin die Substituenten 2.3, 2.4, 2.6 und 2.7 weiter substituiert sein können durch -CN, -Amid oder -Oxim-Funktionen, und 2.5 weiter substituiert sein kann mit durch -CN oder Amid-Funktionen,

oder $R_1$ und $R_2$ bilden zusammen eine Gruppe -O-[($C_1$-$C_6$)-Alkylen]-O-,

$R_3$
1.0H oder
2.0 eine $C_1$-$C_6$-Alkyl-, $C_2$-$C_6$-Alkenyl-, $C_2$-$C_6$-Alkinyl- oder $C_6$-$C_{10}$-Aryl-Gruppe sind, worin Alkyl, Alkenyl und Alkinyl geradkettig oder verzweigt sind, und worin die Alkyl-, Alkenyl- und Alkinyl-Gruppen gegebenenfalls ein- oder zweifach substituiert sind durch:

2.1 -OH,

2.2=O,
2.3-O-$C_1$-$C_6$-Alkyl, worin Alkyl geradkettig oder verzweigt ist,
2.4 -O-$C_2$-$C_6$-Alkenyl, worin Alkenyl geradkettig oder verzweigt ist,
2.5 - $C_6$-$C_{10}$-Aryl,
2.6-NH-$C_1$-$C_6$-Alkyl, worin Alkyl geradkettig oder verzweigt ist,
2.7 -NH-$C_2$-$C_6$-Alkenyl, worin Alkenyl geradkettig oder verzweigt ist,
2.8 -$NH_2$ oder
2.9 Halogen,

und worin die Aryl-Gruppe gegebenenfalls ein- oder zweifach substituiert ist durch Substituent 2.1 oder 2.3 bis 2.9,
worin die Substituenten 2.3, 2.4, 2.6 und 2.7 weiter substituiert sein können durch -CN, -Amid oder -Oxim-Funktionen, und 2.5 weiter substituiert sein kann mit durch -CN oder Amid-Funktionen, und

$R_4$
$CO_2R_3$. $CO_2NHR_3$, CHO, $CH_2OR_3$, $CH_2OSi(R_3)_3$, $CH_2Br$, $CH_2CN$ bedeutet bedeutet, wobei $R_3$ wie oben definiert ist,

oder eine stereoisomere Form der Verbindung der Formel (I) oder ein physiologisch verträgliches Salz der Verbindung der Formel (I) oder ein Salz einer stereoisomeren Form der Verbindung der Formel (I).

2. Verbindung der Formel (I) gemäß Anspruch 1, wobei die Verbindung durch eine Verbindung der Formel (II)

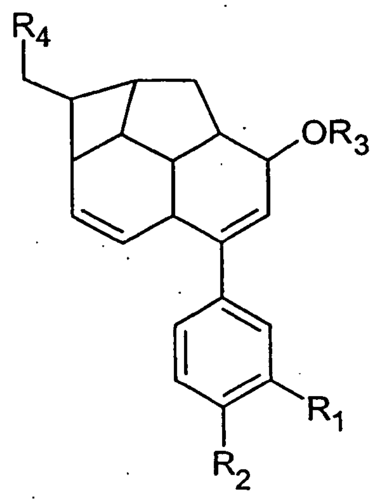

**gekennzeichnet** ist.

3. Verbindung der Formel (I) gemäß Anspruch 1, wobei die Verbindung durch eine Verbindung der Formel (III)

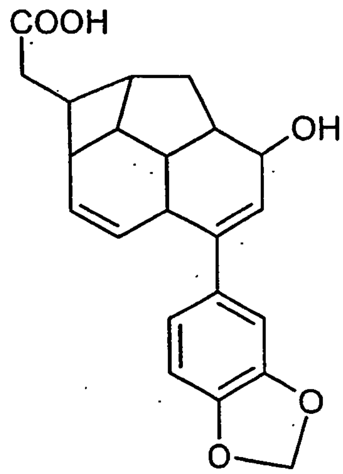

(III)

**gekennzeichnet** ist.

4. Verbindung der Formel (I) gemäß Anspruch 1, wobei die Verbindung durch eine Verbindung der Formel (IV)

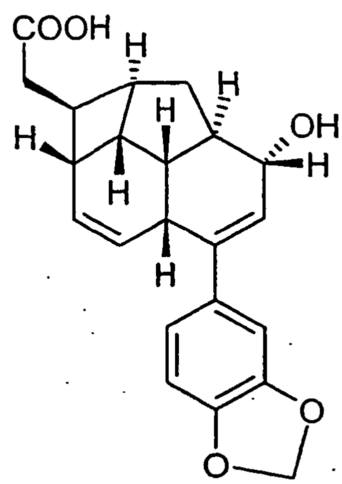

(IV)

**gekennzeichnet** ist.

5. Verfahren zur Herstellung einer Verbindung der Formel (I) gemäß einem oder mehreren der Ansprüche 1 bis 4, **dadurch gekennzeichnet, daß**

1. die Pflanze *Beilschmiedia fulva,* PLA 101037, oder Zellkulturen der Pflanze *Beilschmiedia fulva,* PLA 101037, unter geeigneten Bedingungen extrahiert wird,
2. die Verbindung der Formel (IV) isoliert wird, und
3. gegebenenfalls zu einer Verbindung der Formel (I) derivatisiert und/oder zu einem physiologisch verträglichen Salz der Verbindung der Formel (I) umgesetzt wird.

6. Verfahren zur Herstellung einer Verbindung der Formel (IV) gemäß Anspruch 4, **dadurch gekennzeichnet, daß**

1. die Pflanze *Beilschmiedia fulva,* PLA 101037, oder Zellkulturen der Pflanze *Beilschmiedia fulva,* PLA 101037, unter geeigneten Bedingungen extrahiert wird,
2. die Verbindung der Formel (IV) isoliert wird, und

3. gegebenenfalls zu einem physiologisch verträglichen Salz der Verbindung der Formel (IV) umgesetzt wird.

**7.** Arzneimittel mit einem Gehalt an mindestens einer Verbindung der Formel (I) gemäß einem der Ansprüche 1 bis 4 und einem oder mehreren physiologisch geeigneten Hilfsstoffen.

**8.** Verfahren zur Herstellung eines Arzneimittels gemäß Anspruch 7, **dadurch gekennzeichnet, daß** mindestens einer Verbindung der Formel (I) mit einem oder mehreren physiologisch geeigneten Hilfsstoffen in eine geeignete Darreichungsform gebracht wird.

**9.** Verwendung einer Verbindung der Formel (I) gemäß einem der Ansprüche 1 bis 4 zur Herstellung eines Arzneimittels.

**10.** Verwendung einer Verbindung der Formel (I) gemäß einem der Ansprüche 1 bis 4 zur Herstellung eines Arzneimittels zur Behandlung von allergischen Erkrankungen, von asthmatischen Erkrankungen, von entzündlichen Begleitsymptomen von Asthma und/oder von Krankheiten, die durch Hemmen von c-maf und NFÄT behandelt werden können.

**Claims**

**1.** A compound of the formula (I)

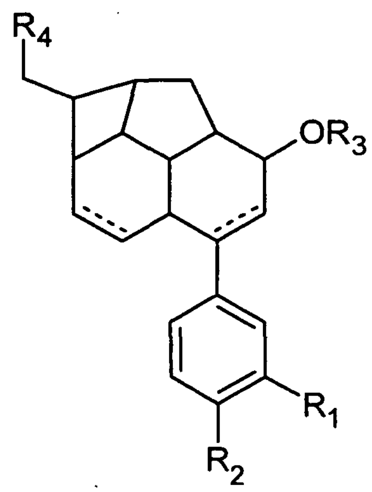

(I)

where

$R_1$ and $R_2$ are, independently of one another,
1.0 H or
2.0 a -O-$C_1$-$C_6$-alkyl, -O-$C_2$-$C_6$-alkenyl, -O-$C_2$-$C_6$-alkynyl or -O-$C_6$-$C_{10}$-aryl group, in which alkyl, alkenyl and alkynyl are straight-chain or branched, and in which the alkyl, alkenyl and alkynyl groups are optionally mono- or disubstituted by:

2.1 -OH,
2.2 =O,
2.3 -O-$C_1$-$C_6$-alkyl in which alkyl is straight-chain or branched,
2.4 -O-$C_2$-$C_6$-alkenyl in which alkenyl is straight-chain or branched,
2.5 $C_6$-$C_{10}$-aryl,
2.6 -NH-$C_1$-$C_6$-alkyl in which alkyl is straight-chain or branched,
2.7 -NH-$C_2$-$C_6$-alkenyl in which alkenyl is straight-chain or branched,
2.8 -$NH_2$ or
2.9 halogen,

and in which the aryl group is optionally mono- or disubstituted by substituent 2.1 or 2.3 to 2.9, in which the substituents 2.3, 2.4, 2.6 and 2.7 may be further substituted by -CN, -amide or -oxime functions, and 2.5 may be further substituted by -CN or amide functions, or

$R_1$ and $R_2$ together form a group -O-[($C_1$-$C_6$)-alkylene]-O-,

$R_3$ is
1.0 H or
2.0 a $C_1$-$C_6$-alkyl, $C_2$-$C_6$-alkenyl, $C_2$-$C_6$-alkynyl or $C_6$-$C_{10}$-aryl group, in which alkyl, alkenyl and alkynyl are straight-chain or branched, and in which the alkyl, alkenyl and alkynyl groups are optionally mono- or disubstituted by:

2.1 -OH,
2.2 =O,
2.3 -O-$C_1$-$C_6$-alkyl in which alkyl is straight-chain or branched,
2.4 -O-$C_2$-$C_6$-alkenyl in which alkenyl is straight-chain or branched,
2.5 -$C_6$-$C_{10}$ aryl,
2.6 -NH-$C_1$-$C_6$-alkyl in which alkyl is straight-chain or branched,
2.7 -NH-$C_2$-$C_6$-alkenyl in which alkenyl is straight-chain or branched,
2.8 -$NH_2$ or
2.9 halogen,

and in which the aryl group is optionally mono- or disubstituted by substituent 2.1 or 2.3 to 2.9, in which the substituents 2.3, 2.4, 2.6 and 2.7 may be further substituted by -CN, -amide or -oxime functions, and 2.5 may be further substituted by -CN or amide functions, and

$R_4$ is
$CO_2R_3$, $CO_2NHR_3$, CHO, $CH_2OR_3$, $CH_2OSi(R_3)_3$, $CH_2Br$, $CH_2CN$, where $R_3$ is as defined above,

or a stereoisomeric form of the compound of the formula (I) or a physiologically tolerated salt of the compound of the formula (I) or a salt of a stereoisomeric form of the compound of the formula (I).

**2.** A compound of the formula (I) as claimed in claim 1, where the compound is **characterized by** a compound of the formula (II)

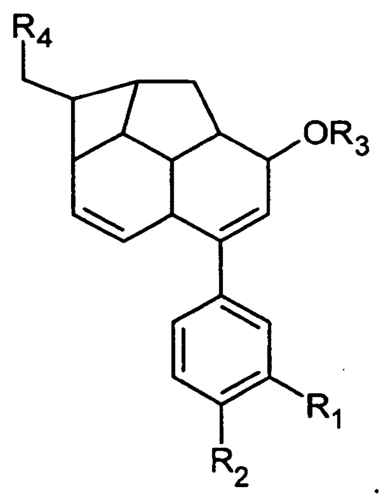

**3.** A compound of the formula (I) as claimed in claim 1, where the compound is **characterized by** a compound of the formula (III)

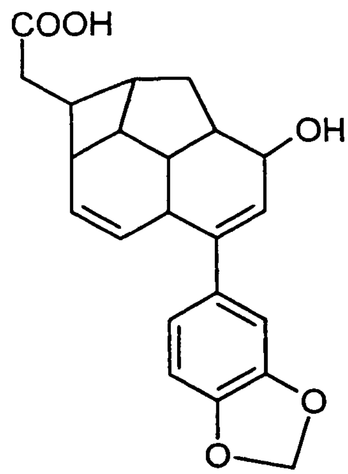

(III).

**4.** A compound of the formula (I) as claimed in claim 1, where the compound is **characterized by** a compound of the formula (IV)

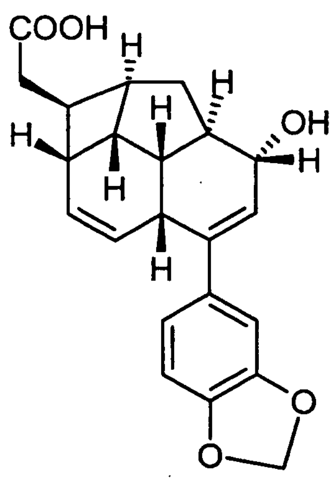

(IV).

**5.** A process for preparing a compound of the formula (I), as claimed in one or more of claims 1 to 4, which comprises

1. extracting the plant *Beilschmiedia fulva,* PLA 101037, or cell cultures of the plant *Beilschmiedia fulva,* PLA 101037, under suitable conditions,
2. isolating the compound of the formula (IV), and
3. where appropriate derivatizing to a compound of the formula (I) and/or reacting to give a physiologically tolerated salt of the compound of the formula (I).

**6.** A process for preparing a compound of the formula (IV) as claimed in claim 4, which comprises

1. extracting the plant *Beilschmiedia fulva,* PLA 101037, or cell cultures of the plant *Beilschmiedia fulva,* PLA 101037, under suitable conditions,
2. isolating the compound of the formula (IV), and
3. where appropriate reacting to give a physiologically tolerated salt of the compound of the formula (IV).

**7.** A medicament having a content of at least one compound of the formula (I) as claimed in any of claims 1 to 4 and of one or more physiologically suitable excipients.

**8.** A process for producing a medicament as claimed in claim 7, which comprises converting at least one compound

of the formula (I) into a suitable dosage form with one or more physiologically suitable excipients.

**9.** The use of a compound of the formula (I) as claimed in any of claims 1 to 4 for producing a medicament.

**10.** The use of a compound of the formula (I) as claimed in any of claims 1 to 4 for producing a medicament for the treatment of allergic disorders, of asthmatic disorders, of inflammatory concomitant symptoms of asthma and/or of diseases which can be treated by inhibiting c-maf and NFAT.

**Revendications**

**1.** Composé de formule (I)

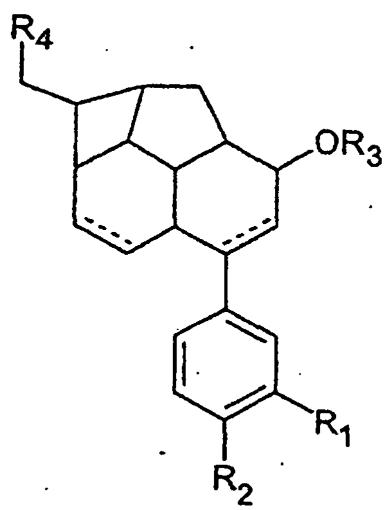

(I)

dans laquelle
$R_1$ et $R_2$ représentent, indépendamment l'un de l'autre,
1.0 H ou
2.0 un groupe -O-alkyle($C_1$-$C_6$), -O-alcényle($C_2$-$C_6$), -O-alcynyle($C_2$-$C_6$) ou -O-aryle ($C_6$-$C_{10}$), les groupes alkyle, alcényle et alcynyle étant à chaîne droite ou ramifiés et les groupes alkyle, alcényle et alcynyle étant éventuellement une ou deux fois substitués par :

2.1 -OH,
2.2 =O,
2.3 -O-alkyle($C_1$-$C_6$), le groupe alkyle étant à chaîne droite ou ramifié,
2.4 -O-alcényle($C_2$-$C_6$), le groupe alcényle étant à chaîne droite ou ramifié,
2.5 -aryle en $C_6$-$C_{10}$,
2.6 -NH-alkyle ($C_1$-$C_6$) le groupe alkyle étant à chaîne droite ou ramifié,
2.7 -NH-alcényle($C_2$-$C_6$), le groupe alcényle étant à chaîne droite ou ramifié,
2.8 -$NH_2$ ou
2.9 halogène,
et le groupe aryle étant éventuellement une ou deux fois substitué par le substituant 2.1 ou 2.3 à 2.9,
les substituants 2.3, 2.4, 2.6 et 2.7 pouvant être encore substitués par -CN, des fonctions -amide ou -oxime, et le substituant 2.5 pouvant être encore substitué par -CN ou des fonctions amide,

ou $R_1$ et $R_2$ forment ensemble un groupe -O-[alkylène ($C_1$-$C_6$)]-O-,
$R_3$ représente
1.0 H ou
2.0 un groupe alkyle en $C_1$-$C_6$, alcényle en $C_2$-$C_6$, -alcynyle en $C_2$-$C_6$ ou aryle en $C_6$-$C_{10}$, les groupes alkyle, alcényle et alcynyle étant à chaîne droite ou ramifiés et les groupes alkyle, alcényle et alcynyle étant éventuellement une ou deux fois substitués par :

2.1 -OH,
2.2 =O,
2.3 -O-alkyle($C_1$-$C_6$), le groupe alkyle étant à chaîne droite ou ramifié,
2.4 -O-alcényle($C_2$-$C_6$), le groupe alcényle étant à chaîne droite ou ramifié,
2.5 -aryle en $C_6$-$C_{10}$,
2.6 -NH-alkyle($C_1$-$C_6$), le groupe alkyle étant à chaîne droite ou ramifié,
2.7 -NH-alcényle($C_2$-$C_6$), le groupe alcényle étant à chaîne droite ou ramifié,
2.8 -$NH_2$ ou
2.9 halogène,

et le groupe aryle étant éventuellement une ou deux fois substitué par le substituant 2.1 ou 2.3 à 2.9,

les substituants 2.3, 2.4, 2.6 et 2.7 pouvant être encore substitués par -CN, des fonctions -amide ou -oxime, et le substituant 2.5 pouvant être encore substitué par -CN ou des fonctions amide, et

$R_4$ représente
$CO_2R_3$, $CO_2NHR_3$ , CHO, $CH_2OR_3$ , $CH_2OSi(R_3)_3$, $CH_2Br$, $CH_2CN$, $R_3$ étant tel que défini ci-dessus, ou forme stéréoisomère du composé de formule (I) ou sel physiologiquement acceptable du composé de formule (I) ou sel d'une forme stéréoisomère du composé de formule (I).

**2.** Composé de formule (I) selon la revendication 1, le composé étant **caractérisé par** un composé de formule (II)

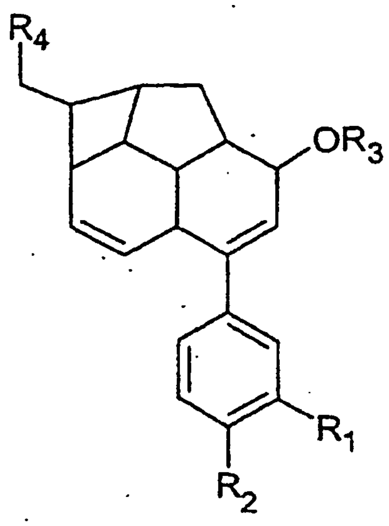

**3.** Composé de formule (I) selon la revendication 1, le composé étant **caractérisé par** un composé de formule (III)

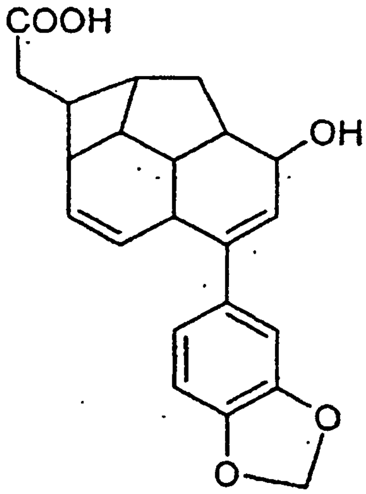

(III)

4. Composé de formule (I) selon la revendication 1, le composé étant **caractérisé par** un composé de formule (IV)

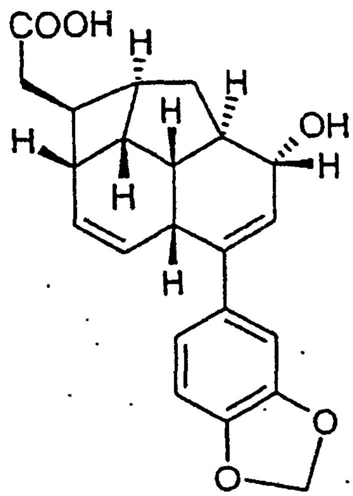

(IV)

5. Procédé pour la préparation d'un composé de formule (I) selon une ou plusieurs des revendications 1 à 4, **caractérisé en ce que**

   1. on extrait dans des conditions appropriées la plante *Beilschmiedia fulva,* PLA 101037 ou des cultures de cellules de la plante *Beilschmiedia fulva,* PLA 101037,
   2. on isole le composé de formule (IV) et
   3. éventuellement on le fonctionnalise en un composé de formule (I) et/ou on le convertit en un sel physiologiquement acceptable du composé de formule (I).

6. Procédé pour la préparation d'un composé de formule (IV) selon la revendication 4, **caractérisé en ce que**

   1. on extrait dans des conditions appropriées la plante *Beilschmiedia fulva,* PLA 101037 ou des cultures de cellules de la plante *Beilschmiedia fulva,* PLA 101037,
   2. on isole le composé de formule (IV) et
   3. éventuellement on le convertit en un sel physiologiquement acceptable du composé de formule (IV).

7. Médicament ayant une teneur en au moins un composé de formule (I) selon l'une quelconque des revendications 1 à 4, et en un ou plusieurs adjuvants physiologiquement appropriés.

**8.** Procédé pour la fabrication d'un médicament selon la revendication 7, **caractérisé en ce qu'**on met sous une forme d'administration appropriée au moins un composé de formule (I) avec un ou plusieurs adjuvants physiologiquement appropriés.

**9.** Utilisation d'un composé de formule (I) selon l'une quelconque des revendications 1 à 4, pour la fabrication d'un médicament.

**10.** Utilisation d'un composé de formule (I) selon l'une quelconque des revendications 1 à 4, pour la fabrication d'un médicament destiné au traitement de maladies allergiques, de maladies asthmatiques, de symptômes inflammatoires accompagnateurs de l'asthme et/ou de maladies qui peuvent être traitées par inhibition de c-maf et NFAT.